(19) Europäisches Patentamt
European Patent Office
Office européen des brevets

(11) Veröffentlichungsnummer : **0 056 119**
**B1**

(12) # EUROPÄISCHE PATENTSCHRIFT

(45) Veröffentlichungstag der Patentschrift : **07.08.85**

(21) Anmeldenummer : **81110342.3**

(22) Anmeldetag : **11.12.81**

(51) Int. Cl.⁴ : **C 07 C 43/29**, C 07 C 43/295, C 07 C 79/35, C 07 C 43/315, C 07 D339/06, C 07 C121/75, C 07 D307/12, C 07 C 59/66, C 07 D317/24, C 07 C 79/46, C 07 C149/41

(54) **Substituierte Diphenylether, diese enthaltende Herbizide und ihre Anwendung als Herbizide.**

(30) Priorität : **09.01.81 DE 3100387**

(43) Veröffentlichungstag der Anmeldung : **21.07.82 Patentblatt 82/29**

(45) Bekanntmachung des Hinweises auf die Patenterteilung : **07.08.85 Patentblatt 85/32**

(84) Benannte Vertragsstaaten :
**AT BE CH DE FR GB IT LI LU NL SE**

(56) Entgegenhaltungen :
**EP-A- 0 025 262**
**FR-A- 2 143 820**
**GB-A- 1 591 960**
**GB-A- 2 025 952**
**US-A- 4 231 953**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Parg, Adolf, Dr.**
**Paray-le-Mondial-Strasse 8**
**D-6702 Bad Duerkheim (DE)**
Erfinder : **Wuerzer, Bruno, Dr. Dipl.-Landwirt**
**Ruedigerstrasse 13**
**D-6701 Otterstadt (DE)**
Erfinder : **Hamprecht, Gerhard, Dr.**
**Rote-Turm-Strasse 28**
**D-6940 Weinheim (DE)**

**EP 0 056 119 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue wertvolle Diphenylether, ihre Anwendung als Herbizide und Herbizide, die diese Verbindungen als Wirkstoffe enthalten.

Es ist bekannt, das Natriumsalz des 2-Chlor-4-trifluormethyl-3'-carboxy-4'-nitrodiphenylethers (A) zur Bekämpfung breitblättriger Unkräuter in Sojabohnen zu verwenden (DE-OS 23 11 638). Unter den bekannten Diphenylethern zeichnet sich der 2-Chlor-4-trifluormethyl-3'-ethoxycarbonyl-methylthio-4'-nitrodiphenylether (C) durch ein hohes Maß an herbizider Aktivität mit geringer Phytotoxizität gegenüber Reis aus (JP-OS 77/21320). Ferner sind die herbiziden Eigenschaften des 2-Chlor-4-trifluormethyl-3'-methyl-4'-nitrodiphenylethers (B) bekannt (DE-OS 23 04 006).

Es wurde nun gefunden, daß die neuen substituierten Diphenylether der allgemeinen Formel I

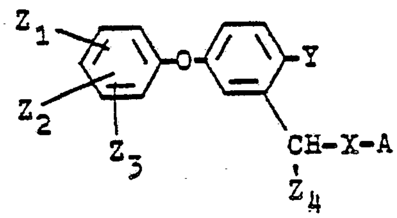

(I)

in der

$Z_1$ 2-Chlor,
$Z_2$ 4-Trifluormethyl,
$Z_3$ Wasserstoff,
$Z_4$ Wasserstoff, Cyan, $C_1$-$C_4$-Alkyl,
$C_1$-$C_4$ Alkoxy, Acetoxy oder $C_1$-$C_4$ Alkylmercapto bedeutet,
Y Nitro,
X Sauerstoff, Schwefel, Sulfinyl oder Sulfonyl bedeutet,

und

A $C_1$-$C_6$ Alkyl,
substituiertes $C_1$-$C_4$ Alkyl

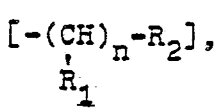

Tetrahydrofurfuryl,
alkyl($C_1$-$C_4$) substituiertes Tetrahydrofurfuryl,
Phenyl,
substituiertes Phenyl

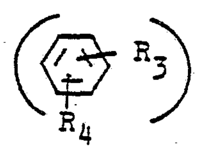

Benzyl,
substituiertes Benzyl

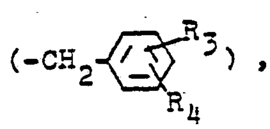

Acyl

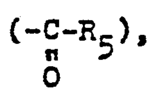

Silyl

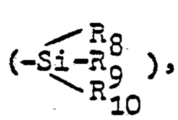

oder Phosphatyl

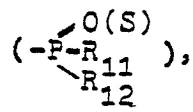

bedeutet, wobei im Falle X = Sauerstoff A zusätzlich Sulfonyl ($—SO_2R_{13}$) oder Sulfamoyl

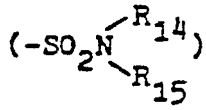

bedeutet und im Falle $Z_4$ = Alkoxy oder Alkylmercapto A zusätzlich eine Methylenkette $—(CH_2)_m—$ bedeutet, durch die die Reste $Z_4—CH—X—$ zu einem Ring verbunden werden, $R_1$ Wasserstoff, Methyl, Ethyl oder n-Propyl, $R_2$ Cyan, Methoxy, Ethoxy oder

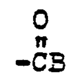

bedeutet, wobei B OH, ONa, $OCH_3$, $OC_2H_5$, $OC_3H_7(i)$ oder O-Alkyl $C_4$-$C_{20}$, O-Phenyl und substituiertes Phenoxy, $NH_2$, —NH-Alkyl $C_1$-$C_4$, —N(Alkyl $C_1$-$C_4)_2$ bedeutet, n bedeutet 1, 2 oder 3, $R_3$ und $R_4$ bedeuten unabhängig voneinander Wasserstoff, Halogen, Methyl, Nitro, Cyan, Methoxy, Carboxyl, Trifluormethyl oder O-Propionsäuremethylester, $R_5$ bedeutet Methyl, Ethyl, Propyl, Chlormethyl, Dichlormethyl, Trichlormethyl, Methoxymethyl, Phenyl sowie Nitro- oder alkyl($C_1$-$C_4$) substituiertes Phenyl oder alpha-2,4-Dichlorphenoxyethyl, $R_8$, $R_9$ und $R_{10}$ bedeuten Methyl, Ethyl, n-Propyl oder n-Butyl und können gleich oder verschieden sein, $R_{11}$ und $R_{12}$ bedeuten Methoxy, Ethoxy, Thiomethyl, Thioethyl, Thio-n-propyl, N,N-Dimethylamino oder N,N-Diethylamino und können gleich oder verschieden sein, $R_{13}$ bedeutet Methyl, Ethyl, Phenyl oder Trifluormethyl, $R_{14}$ und $R_{15}$ bedeuten Wasserstoff, Methyl, Ethyl, n-Propyl, iso-Propyl, n-Butyl, n-Hexyl, n-Heptyl, Methoxy, Ethoxy oder Isopropyloxy und können gleich oder verschieden sein, m bedeutet 2 oder 3, eine hervorragende herbizide Wirkung haben und von einer Reihe von Kulturpflanzen toleriert werden.

$Z_4$ kann beispielsweise für Wasserstoff, Cyan, Methyl, Ethyl, n-Propyl, iso-Propyl, n-Butyl, iso-Propyl, iso-Butyl, tert.-Butyl, Methoxy, Ethoxy, Propoxy, Butoxy, Methylmercapto, Ethylmercapto oder Propylmercapto, X beispielsweise für Sauerstoff, Schwefel, Sulfinyl oder Sulfonyl stehen und A kann beispielsweise folgende Bedeutungen haben : Methyl, Ethyl, n-Propyl, 2-Propyl, n-Butyl, 2-Butyl, 3-Butyl, 2,2-Dimethylethyl, n-Pentyl und alle isomeren Pentylreste, n-Hexyl und alle isomeren Hexylreste, substituierte $C_1$-$C_4$-Alkylreste

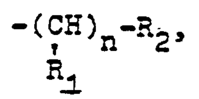

wobei $R^1$ für Wasserstoff, Methyl, Ethyl oder n-Propyl, $R_2$ für Cyan, Methoxy, Ethoxy oder

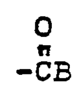

steht und B für OH, ONa, $OCH_3$, $OC_2H_5$, $OC_3H_7(i)$ oder O-Alkyl $C_4$-$C_{20}$, O-Phenyl und substituiertes Phenoxy, $—NH_2$, —NH-Alkyl($C_1$-$C_4$) oder —N(Alkyl $C_1$-$C_4)_2$ steht, und n die Zahlen 1, 2 oder 3 bedeutet ; A bedeutet ferner Tetrahydrofurfuryl oder alkyl($C_1$-$C_4$)substituiertes Tetrahydrofurfuryl ; Phenyl, substituiertes Phenyl

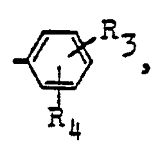

wobei $R_3$ und $R_4$ für Wasserstoff, Halogen (Fluor, Chlor, Brom), Methyl, Nitro, Cyan, Methoxy, Carboxy, Trifluormethyl oder O-Propionsäuremethylester, stehen und gleich oder verschieden sein können ; Benzyl, substituiertes Benzyl

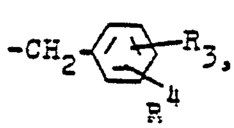

wobei $R_3$ und $R_4$ die oben genannten Bedeutungen besitzen ; Acyl

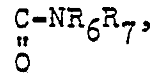

wobei $R_5$ für Methyl, Ethyl, Propyl, Chlormethyl, Dichlormethyl, Trichlormethyl, Methoxymethyl, Phenyl, alkyl($C_1$-$C_4$) substituiertes Phenyl oder α-2,4-Dichlorphenoxyethyl steht ; Silyl

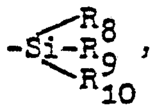

wobei $R_8$, $R_9$ und $R_{10}$ für Methyl, Ethyl, n-Propyl oder n-Butyl stehen und gleich oder verschieden sein können ; Phosphatyl

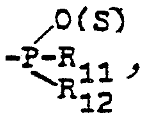

wobei $R_{11}$ und $R_{12}$ für Methoxy, Ethoxy, Thiomethyl, Thioethyl, Thio-n-propyl, N,N-Dimethylamino oder N,N-Diethylamino stehen und gleich oder verschieden sein können ; im Falle X = Sauerstoff bedeutet A zusätzlich die Reste Sulfonyl $-SO_2R_{13}$, wobei $R_{13}$ für Methyl, Ethyl, Phenyl oder Trifluormethyl steht ; und Sulfamoyl $-SO_2NR_{14}R_{15}$, wobei $R_{14}$ und $R_{15}$ für Wasserstoff, Methyl, Ethyl, n-Propyl, iso-Propyl, n-Butyl, n-Hexyl, n-Heptyl, Methoxy, Ethoxy oder Isopropyloxy stehen und gleich oder verschieden sein können ; und im Falle $Z_4$ = Alkoxy oder Alkylmercapto A zusätzlich eine Methylenkette $-(CH_2)_m-$ bedeutet, durch die die Reste $Z_4-CH-X-$ zu einem Ring verbunden werden, wobei m = 2 oder 3 bedeutet.

Niedere Alkylreste sind Alkylreste mit 1 bis 4 C-Atomen.

Die Herstellung der neuen Verbindungen der Formel I kann beispielsweise aus den folgenden Formelschemata ersehen werden :

Formelschema a)

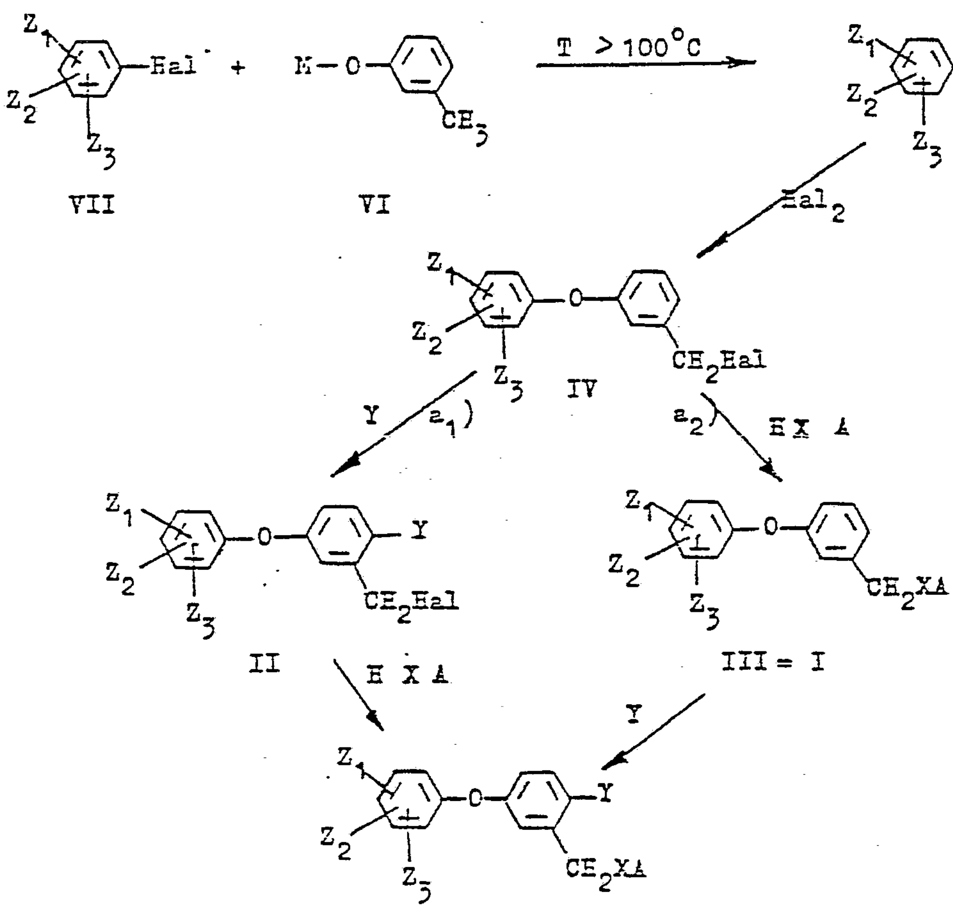


VII VI V IV II III = I I

In diesen Formeln haben $Z_1$, $Z_2$, $Z_3$, X, Y und A die unter Formel I angegebene Bedeutung und Hal bezeichnet ein Halogenatom, vorzugsweise Chlor oder Brom, und M steht für ein Metallkation, vorzugsweise Natrium oder Kalium. Man kondensiert ein substituiertes Halogenbenzol der allgemeinen Formel VII mit dem Alkalisalz des meta-Kresols der allgemeinen Formel VI in einem polar aprotischen Lösungsmittel, z. B. Dimethylsulfoxid, Dimethylformamid oder N-Methylpyrrolidon, bei erhöhter Temperatur (80 °C bis 200 °C) und erhält in guten Ausbeuten den entsprechenden 3-Methyldiphenylether der allgemeinen Formel V. Die Überführung in die Halogenmethylverbindung kann in sehr einfacher und kostengünstiger Arbeitsweise mittels direkter Halogenierung, gegebenenfalls in einem inerten Lösungsmittel und unter Zusatz von Radikalstartern und Einwirkung von Licht (Houben Weyl Band 5/3, Georg Thieme Verlag, Stuttgart 1962, Seite 736 ; DOS 28 44 270), oder mit Hilfe von Halogenierungsagentien wie Sulfurylchlorid oder N-Bromsuccinimid in einem inerten Lösungsmittel und unter Zusatz von Radikalstartern (Houben Weyl Band 5/3, Georg Thieme Verlag, Stuttgart, 1962, Seite 892, Band 5/4, Seite 341, 1960) erfolgen.

Die Herstellung der erfindungsgemäßen Endstoffe der allgemeinen Formel I ist beispielsweise über zwei Wege möglich.

Bei Reaktionsweg $a_1$) wird zunächst der jeweilige Substituent Y (Y verschieden von H) nach allgemein bekannten Verfahren wie Nitrierung (Salpetersäure/Schwefelsäure) oder Halogenierung eingeführt und man erhält in guten Ausbeuten die entsprechend substituierte Halogenmethylverbindung der allgemeinen Formel II. Die weitere Umsetzung mit Alkohol-bzw. Thiolkomponenten HXA in Gegenwart eines säurebindenden Mittels oder in Form ihrer Alkalimetallsalze und in einem inerten Lösungsmittel liefert die Endstoffe der allgemeinen Formel I.

Im Reaktionsweg $a_2$) erfolgt zunächst, wie bei $a_1$) beschrieben, die Umsetzung der Halogenmethylverbindung IV mit den Alkohol- bzw. Thiolkomponenten zu der Benzyletherverbindungen der allgemeinen Formel III, die gleichzeitig identisch sind für die Endstoffe mit Y = H. Durch Einführung des Substituenten Y erhält man schließlich wie angegeben, die Endstoffe der allgemeinen Formel I.

(Siehe Schema Seite 6 f.)

Formelschema b)

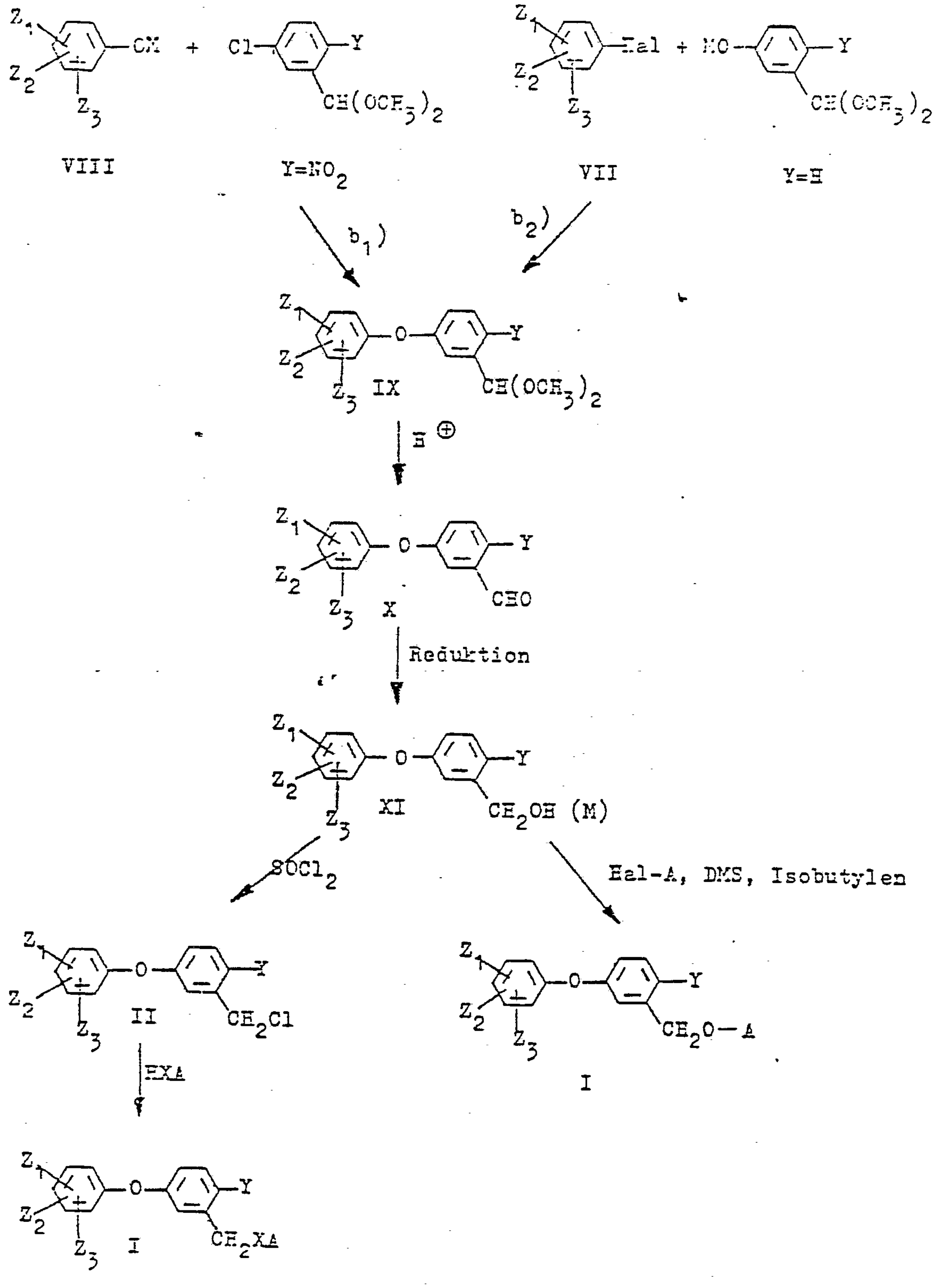


In diesen Formeln haben $Z_1$, $Z_2$, $Z_3$, X, Y und A die unter Formel I angegebene Bedeutung und Hal bezeichnet ein Halogenatom, vorzugsweise Chlor oder Brom und M steht für ein Metallkation, vorzugsweise Natrium oder Kalium.

Die Darstellung des Benzylalkohols der allgemeinen Formel XI, dem Ausgangspunkt für die Synthese der gewünschten Endprodukte der allgemeinen Formel I, ist über zwei Verfahrenswege möglich. Im Verfahren $b_1$) kondensiert man das Alkalisalz der entsprechend substituierten Phenole mit dem 5-Chlor-2-nitrobenzaldehyl-dimethylacetal (Journal of American Chemical Society *74*, 536 (1952)) in einem polaren, aprotischen Lösungsmittel, wie z. B. Dimethylsulfoxid, Dimethylformamid oder N-Methylpyrrolidon, bei erhöhter Temperatur (80 bis 200 °C) und erhält in guten Ausbeuten das entsprechende Diphenyletherbenzaldehyddimethylacetal der allgemeinen Formel IX mit $Y = NO_2$. Im Verfahren $b_2$) liefert die Reaktion des Halogenbenzols der allgemeinen Formel VII mit dem Alkalisalz des 3-Hydroxybenzaldehyddimethylacetals unter den gleichen Reaktionsbedingungen wie bei $b_1$) das Diphenyletherbenzaldehyddimethylacetal IX mit Y = H. Y kann jedoch durch Nitrierung mit Salpetersäure in Eisessig bei 0 °C bis 5 °C in die Nitrogruppe oder durch Halogenierung mit elementarem Halogen in Eisessig in ein Halogenatom überführt werden. Zum Benzylalkohol mit der allgemeinen Formel XI gelangt man durch saure Hydrolyse des Acetals mit konzentrierter Mineralsäure und anschließender Reduktion des Aldehyds X mit einem Reduktionsmittel wie Natriumborhydrid oder Aluminiumisopropylat in einem inerten Lösungsmittels. Die Endprodukte der allgemeinen Formel I können nun aus dem Benzylalkohol XI direkt durch Umsetzung mit Acylierungs- oder Alkylierungskomponenten nach jeweils allgemein literaturbekannten Methoden gewonnen werden, wobei der Benzylalkohol auch in Form seines Alkalimetallsalzes zur Reaktion gebracht werden kann. Mittels Thionylchlorid oder anderer chorierender Agentien kann der Benzylalkohol XI auch in das Benzylchlorid II überführt werden, aus dem sich die Endprodukte der allgemeinen Formel I analog Formelschema a) gewinnen lassen.

Der Vorteil der hier angegebenen Verfahrensweisen zur Herstellung der herbizid wirksamen Diphenylether der allgemeinen Formel I liegt in dem Einsatz billiger, leicht zugänglicher Einsatzstoffe (besonders bei Verfahrensweise a) und in den hohen Ausbeuten der einzelnen Reaktionsschritte. Ferner können durch die chemische Variabilität der einzelnen Zwischenprodukte eine Vielzahl, in ihren biologischen Eigenschaften unterschiedlicher Endstoffe synthetisiert werden.

Die folgenden Beispiele erläutern die Herstellung der neuen Verbindungen der allgemeinen Formel I nach den angegebenen Verfahren. Gewichtsteile verhalten sich zu Volumenteilen wie kg zu Liter.

## Beispiel 1

Eine Lösung von 19 Gew.-Teilen 3-(2'-Chlor-4'-trifluormethylphenoxy)-benzyloxyessigsäuremethylester in 60 Volumenteilen Essigsäureanhydrid werden bei 0 bis 5 °C mit einem Gemisch aus 6,2 Gew.-Teilen 65 %iger Salpetersäure und 6,2 Gew.-Teilen konzentrierter Schwefelsäure nitriert. Anschließend wird 2 Stunden bei 5 °C nachgerührt, die Reaktionsmischung in 500 Volumenteilen Wasser eingerührt und zweimal mit je 200 Volumenteilen Methylenchlorid extrahiert. Die organische Phase wird mit Magnesiumsulfat getrocknet, abfiltriert und unter Vakuum eingeengt. Der ölige Rückstand wird über Kieselgel mit Aceton/Toluol (30 : 70) chromatographiert. Man erhält 22 Gew.-Teile (= 52 % d. Th.) 3-(2'-Chlor-4'-trifluormethylphenoxy)-6-nitro-benzyloxyessigsäuremethylester (Verbindung Nr. 5) mit dem Brechungsindex $n_D^{25}$ : 1,547 2.

## Beispiel 2

Eine Lösung von 346 Gew.-Teilen 3-(2'-Chlor-4'-trifluormethylphenoxy)-benzaldehyddimethylacetal in 600 Volumenteilen Essigsäureanhydrid wird bei 0 bis 5 °C mit einer Mischung aus 124 Gew.-Teilen 65 %iger Salpetersäure und 128 Gew.-Teilen konzentrierter Schwefelsäure tropfenweise versetzt. Man rührt 2 Stunden bei 5 °C nach und trägt die Reaktionsmischung dann in 5 000 Gew.-Teilen Eis ein. Der Niederschlag wird abgesaugt, getrocknet, mit Methyl-tertiärbutylether verrührt und erneut abgesaugt. Man erhält 380 Gew.-Teile (= 85 % d. Th.) 3-(2'-Chlor-4'-trifluormethylphenoxy)-6-nitro-benzaldehyddiacetat (Verbindung Nr. 9) mit dem Schmelzpunkt 108 bis 111 °C.

## Beispiel 3

a) Aus 447 Gew.-Teilen 3-(2'-Chlor-4'-trifluormethylphenoxy)-6-nitro-benzaldehyd-diacetat erhält man durch saure Hydrolyse 330 Gew.-Teile (= 96 % d. Th.) 3-(2'-Chlor-4'-trifluormethylphenoxy)-6-nitrobenzaldehyd mit dem Schmelzpunkt 94 bis 96 °C (aus Diisopropylether umkristallisiert).

b) 17,3 Gew.-Teile 3-(2'-Chlor-4'-trifluormethylphenoxy)-6-nitrobenzaldehyd und 4,7 Gew.-Teile Ethandithiol werden in 100 Volumenteilen absolutem Toluol 3 Stunden unter Rückfluß gerührt. Das Reaktionsgemisch wird unter Vakuum eingeengt und man erhält 21 Gew.-Teile (= 100 % d. Th.) 3-(2'-Chlor-4'-trifluormethylphenoxy)-6-nitro-benzaldehyd-1,3-dithiolan (Verbindung Nr. 10) mit dem Schmelzpunkt 68 bis 72 °C.

## Beispiel 4

a) Aus 34,6 Gew.-Teilen 3'-(2'-Chlor-4'-trifluormethylphenoxy)-6-nitro-benzaldehyd durch Reduktion mit Natriumborhydrid erhält man 22 Gew.-Teile (= 63 % d. Th.) 3'-(2'-Chlor-4'-trifluormethylphenoxy)-6-nitrobenzylalkohol mit dem Schmelzpunkt 89 bis 92 °C (aus Diisopropylether umkristallisiert).

b) Zu einer Lösung von 17,4 Gew.-Teilen 3-(2'-Chlor-4'-trifluormethylphenoxy)-6-nitro-benzylalkohol in 150 Volumenteilen absolutem Tetrahydrofuran werden gleichzeitig 7,8 Gew.-Teile Isopropylamidosulfonylchlorid und 5,1 Gew.-Teile Triethylamin zugetropft. Anschließend wird die Reaktionsmischung 2 Stunden bei Rückflußtemperatur nachgerührt. Nach dem Abkühlen saugt man den Niederschlag ab und engt das Filtrat unter Vakuum ein. Der ölige Rückstand wird in 50 Volumenteilen 1 n-Natronlauge verrührt, filtriert und mit 2 n Salzsäure bis pH = 2 versetzt. Der Niederschlag wird abgesaugt, getrocknet und man erhält 18 Gew.-Teile (= 90 % d. Th.) 3-(2'-Chlor-4'-trifluormethylphenoxy)-6-nitro-benzyloxy-N-isopropylamidosulfonat (Verbindung Nr. 12) mit dem Schmelzpunkt 89 bis 91 °C.

Beispiel 5

a) 34,8 Gew.-Teile 3-(2'-Chlor-4'-trifluormethylphenoxy)-6-nitro-benzylalkohol werden in 200 Volumenteilen absolutem Toluol suspendiert, mit 8 Gew.-Teilen Pyridin versetzt und bei 0 °C 13,1 Gew.-Teile Thionylchlorid zugetropft. Danach rührt man die Reaktionsmischung 2 Stunden bei 110 °C nach, kühlt ab und extrahiert das Reaktionsgemisch einmal mit 75 Volumenteilen 3 n Salzsäure und fünfmal mit 250 Volumenteilen Wasser. Die organische Phase wird abgetrennt, mit Magnesiumsulfat getrocknet und unter Vakuum eingeengt. Man erhält 32 Gew.-Teile (= 88 % d. Th.) 3-(2'-Chlor-4'-trifluormethylphenoxy)-6-nitro-benzylchlorid in Form eines zunächst öligen Rückstandes, der durch Verreiben mit Pentan kristallisiert und den Schmelzpunkt 55 bis 60 °C besitzt.

b) 1,44 Gew.-Teile Natriumhydrid (80 %ig) werden in 25 Volumenteilen absolutem Tetrahydrofuran suspendiert und bei Raumtemperatur mit einer Lösung von 5,31 Gew.-Teilen Mercaptoessigsäuremethylester in 50 Volumenteilen absolutem Tetrahydrofuran portionsweise versetzt. Man rührt eine halbe Stunde nach und setzt dann eine Lösung von 18,3 Gew.-Teilen 3-(2'-Chlor-4'-trifluormethylphenoxy)-6-nitro-benzylchlorid in 50 Volumenteilen absolutem Tetrahydrofuran zu. Die Reaktionsmischung wird anschließend 3 Stunden bei Rückflußtemperatur nachgerührt, abgekühlt und unter Vakuum eingeengt. Der Rückstand wird in Ether aufgenommen, mit Wasser extrahiert, die organische Phase abgetrennt, mit Magnesiumsulfat getrocknet, abfiltriert und der Ether verdampft. Der ölige Rückstand kristallisiert bei Anreiben und wird aus Diisopropylether umkristallisiert. Man erhält 16 Gew.-Teile (73 % d. Th.) 3-(2'-Chlor-4'-trifluormethylphenoxy)-6-nitro-benzyl-thiolessigsäuremethylester (Verbindung Nr. 13) mit dem Schmelzpunkt 61 bis 63 °C.

Beispiel 6

Eine Lösung von 17,4 Gew.-Teilen 3-(2'-Chlor-4'-trifluormethylphenoxy)-6-nitro-benzylthiolessigsäuremethylester in 200 Volumenteilen Chloroform wird bei − 10 °C mit einer Lösung von 19,5 Gew.-Teilen meta-Chlorperbenzoesäure in 200 Volumenteilen Chloroform portionsweise versetzt. Man läßt die Reaktionsmischung 48 Stunden bei 5 °C stehen und saugt den gebildeten Niederschlag ab. Das Filtrat wird mit verdünnter, wäßriger Natriumbicarbonatlösung und Wasser extrahiert, mit Magnesiumsulfat getrocknet, abfiltriert und unter Vakuum eingedampft. Der Rückstand wird mit Pentan verrieben und man erhählt 12 Gew.-Teile (= 64 % d. Th.) 3-(2'-Chlor-4'-trifluormethylphenoxy)-6-nitro-benzyl-sulfonessigsäuremethylester (Verbindung Nr. 14) mit dem Schmelzpunkt von 82 bis 83 °C.

Beispiel 7

16 Gew.-Teile 3-(2'-Chlor-4'-trifluormethylphenoxy)-6-nitrobenzylthiolessigsäuremethylester werden in einer Lösung von 2,5 Gew.-Teilen kaliumhydroxid in 15 Volumenteilen Ethanol und 15 Volumenteilen Wasser 3 Stunden bei Rückflußtemperatur gehalten. Man kühlt ab, verdünnt mit 100 Volumenteilen Wasser und säuert die Reaktionsmischung mit 2 n Salzsäure an. Der ölige Rückstand wird mit Methylenchlorid aufgenommen, mit Magnesiumsulfat getrocknet, abfiltriert und unter Vakuum das Lösungsmittel abgedampft. Der Rückstand wird über Kieselgel mit Toluol/Aceton (70 : 30) chromatographiert. Man erhält 13,4 Gew.-Teile (= 86 % d. Th.) 3-(2'-Chlor-4'-trifluormethylphenoxy)-6-nitro-benzyl-thiolessigsäure (Verbindung Nr. 15) mit dem Brechungsindex $n_D^{25}$ : 1,582 1.

Beispiel 8

In eine Lösung von 17,4 Gew.-Teilen 3-(2'-Chlor-4'-trifluormethylphenoxy)-6-nitro-benzylalkohol in 100 Volumenteilen 1,2-Dichlorethan und 0,5 Gew.-Teilen Schwefelsäure werden bei 40 °C zwei Stunden Isobutylen eingegast. Das Reaktionsgemisch wird dann mit verdünnter, wäßriger Natriumbicarbonatlösung und zweimal mit Wasser gewaschen, die organische Phase mit Magnesiumsulfat getrocknet, abfiltriert und eingeengt. Man erhält 18 Gew.-Teile (= 89 % d. Th.) 3-(2'-Chlor-4'-trifluormethylphenoxy)-6-nitro-benzyl-tertiär-butylether (Verbindung Nr. 16) mit dem Schmelzpunkt 75 bis 78 °C.

Beispiel 9

18,3 Gew.-Teile 3-(2'-Chlor-4'-trifluormethylphenoxy)-6-nitro-benzylchlorid in 100 Volumenteilen absolutem Aceton werden bei Raumtemperatur mit einer Lösung von O-Ethyl-S-propyl-dithiophosphorsäureester-dimethylammoniumsalz in 100 Volumenteilen Aceton versetzt. Man erhitzt das Reaktionsgemisch 7 Stunden auf Rückflußtemperatur, kühlt dann ab und engt unter Vakuum zur Trockene ein. Der Rückstand wird in Toluol aufgenommen, mit verdünnter, wäßriger Natriumbicarbonatlösung und mit Wasser extrahiert, mit Magnesiumsulfat getrocknet und unter Vakuum eingeengt. Man erhält 14 Gew.-Teile (= 52 % d. Th.) O-Ethyl-S-propyl-S-[3-(2'-Chlor-4'-trifluormethylphenoxy)-6-nitrobenzyl]-dithiophosphorsäureester (Verbindung Nr. 17) mit dem Brechungsindex $n_D^{25}$ : 1,566 6.

Beispiel 10

Eine Suspension von 17,3 Gew.-Teilen 3-(2'-Chlor-4'-trifluormethylphenoxy)-6-nitrobenzaldehyd, 22,5 Gew.-Teilen einer 40 %igen Natriumhydrogensulfitlösung und 63 Volumenteilen Wasser wird zunächst eine halbe Stunde bei 40 bis 50 °C gerührt, auf 0 °C abgekühlt, mit 200 Volumenteilen Ether und dann mit einer Lösung von 4,88 Gew.-Teilen Kaliumcyanid in 63 Volumenteilen Wasser versetzt. Nach Zugabe einer Spatelspitze Triethylbenzylammoniumchlorid wird zunächst 2 Stunden bei Raumtemperatur und dann 2 Stunden bei Rückfluß nachgerührt. Man kühlt ab, separiert die Etherphase und wäscht diese mit verdünnter, wäßriger Natriumbicarbonatlösung und Wasser, trocknet mit Magnesiumsulfat und zieht unter Vakuum das Lösungsmittel ab. Man erhält 10 Gew.-Teile (54 % d. Th.) 3-(2'-Chlor-4'-trifluormethylphenoxy)-6-nitro-α-cyano-benzylalkohol (Verbindung Nr. 18) mit dem Brechungsindex $n_D^{25}$ : 1,565 0.

(Siehe Tabelle Seite 10 ff.)

| Verbindung Nr. | 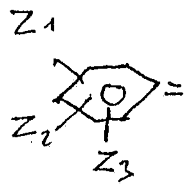 = D | Y | $Z_4$ | X | A | Fp [$^oC$] $n_D^{25}$, Wellenlänge einer Bande im IR-Spektrum |
|---|---|---|---|---|---|---|
| 87 | 2'-Chlor-4'-trifluormethylphenyl | $NO_2$ | H | O | $CH_3$ | 80- 85 |
| 88 | " | " | CN | " | $CH_3$ | |
| 92 | " | $NO_2$ | H | S | $CH_3$ | |
| 93 | " | " | " | S=O | $CH_3$ | |
| 94 | " | " | " | $SO_2$ | $CH_3$ | 110-113 |
| 95 | " | " | " | O | $C_2H_5$ | |
| 96 | " | " | " | S | $CH_2CH_2CN$ | C=N = 2240 |
| 97 | " | " | " | " | Benzyl | |
| 98 | " | " | " | S=O | Benzyl | |
| 99 | " | " | " | $SO_2$ | Benzyl | |
| 100 | " | " | " | O | 4-Chlorbenzyl | |
| 101 | " | " | " | S | 4-Chlorbenzyl | 1,6142 |

| Verbindung Nr. | D | Y | $Z_4$ | X | A | Fp [°C] $n_D^{25}$, Wellenlänge einer Bande im IR-Spektrum |
|---|---|---|---|---|---|---|
| 104 | 2'-Chlor-4'-trifluormethylphenyl | $NO_2$ | H | O | $CH_2$- 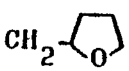 | 1,5360 |
| 109 | " | " | " | O | $CH_2COOC_2H_5$ | |
| 110 | " | " | " | " | $CH_2COOH$ | |
| 111 | " | " | " | " | $CH_2COOC_{18}H_{37}$ | |
| 112 | " | " | " | " | $CH_2COONa$ | |
| 113 | " | " | " | " | $CH_2COO-C_3H_7(i)$ | |
| 114 | " | " | " | " | $CH_2COO$-phenyl | |
| 115 | " | " | " | " | $CH_2COO$-3-F-phenyl | |

| Verbindung Nr. | D | Y | $Z_4$ | X | A | Fp [°C] $n_D^{25}$, Wellenlänge einer Bande im IR-Spektrum |
|---|---|---|---|---|---|---|
| 120 | 2'-Chlor-4'-trifluormethylphenyl | $NO_2$ | H | O | $CH-COOCH_3$<br>$\vert$<br>$CH_3$ | 1,5345 |
| 121 | " | " | " | " | $CH-COOH$<br>$\vert$<br>$CH_3$ | |
| 122 | " | " | " | " | $CH-COO-Na$<br>$\vert$<br>$CH_3$ | |
| 124 | " | " | " | S | 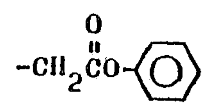 | |
| 125 | " | " | " | " | 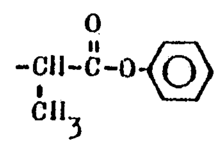 | |

| Verbindung Nr. | D | Y | $Z_4$ | X | A | Fp [°C] $n_D^{25}$, Wellenlänge einer Bande im IR-Spektrum |
|---|---|---|---|---|---|---|
| 131 | 2'-Chlor-4'-trifluormethylphenyl | $NO_2$ | H | S | $-\underset{C_2H_5}{CH}-\overset{O}{\overset{\Vert}{C}}OH$ | |
| 132 | " | " | " | " | $-\underset{C_2H_5}{CH}-\overset{O}{\overset{\Vert}{C}}OCH_3$ | |
| 133 | " | " | " | " | $-\underset{C_2H_5}{CH}-\overset{O}{\overset{\Vert}{C}}OC_2H_5$ | |
| 134 | " | " | " | " | $-\underset{C_2H_5}{CH}-\overset{O}{\overset{\Vert}{C}}OC_{12}H_{25}$ | |

| Verbindung Nr. | D | Y | $Z_4$ | X | A | Fp [°C] $n_D^{25}$, Wellenlänge einer Bande im IR-Spektrum |
|---|---|---|---|---|---|---|
| 135 | 2'-Chlor-4'-trifluormethylphenyl | $NO_2$ | H | S | $-CH(C_2H_5)-COOC_{18}H_{37}$ | |
| 136 | " | " | " | " | $-CH_2CH_2CH_2COOH$ | |
| 137 | " | " | " | " | $-CH_2CH_2CH_2COOCH_3$ | |
| 138 | " | " | " | " | $-CH_2CH_2CH_2COOC_2H_5$ | |
| 140 | " | " | " | S | $CH(CH_3)-COOCH_3$ | 1,5541 |
| 141 | " | " | " | " | $CH(CH_3)-COOC_{12}H_{25}$ | |
| 142 | " | " | " | " | $CH(CH_3)-COOH$ | 1,5636 |

| Verbindung Nr. | D | Y | $Z_4$ | X | A | Fp [°C] $n_D^{25}$, Wellenlänge einer Bande im IR-Spektrum |
|---|---|---|---|---|---|---|
| 144 | 2'-Chlor-4'-trifluormethylphenyl | $NO_2$ | H | S | $CH_2CH_2COOCH_3$ | |
| 145 | " | " | " | " | $CH_2CH_2COOC_{12}H_{25}$ | 1,5188 |
| 146 | " | " | " | " | $CH_2CH_2COOC_{18}H_{37}$ | |
| 147 | " | " | " | " | $CH_2CH_2COOH$ | |
| 148 | " | " | " | S=O | $CH_2COOCH_3$ | 98-100 |
| 149 | " | " | " | " | $CH_2COOH$ | 144-146 |
| 150 | " | " | " | " | $CH-COOCH_3$<br>$\vert$<br>$CH_3$ | |
| 151 | " | " | " | " | $CH-COOH$<br>$\vert$<br>$CH_3$ | |
| 152 | " | " | " | $SO_2$ | $CH_2COOCH_3$ | 80- 81 |
| 153 | " | " | " | " | $CH_2COOH$ | 95- 97 |
| 154 | " | " | " | " | $CH-COOCH_3$<br>$\vert$<br>$CH_3$ | |
| 155 | " | " | " | " | $CH-COOH$<br>$\vert$<br>$CH_3$ | |

| Verbindung Nr. | D | Y | $Z_4$ | X | A | Fp [°C] $n_D^{25}$, Wellenlänge einer Bande im IR-Spektrum |
|---|---|---|---|---|---|---|
| 172 | 2'-Chlor-4'-trifluormethylphenyl | $NO_2$ | H | O | Phenyl | |
| 173 | " | " | " | " | 4-F-phenyl | $NO_2$ = 1560 |

| Verbindung Nr. | D | Y | $Z_4$ | X | A | Fp [°C] $n_D^{25}$, Wellenlänge einer Bande im IR-Spektrum |
|---|---|---|---|---|---|---|
| 174 | 2'-Chlor-4'-trifluormethylphenyl | $NO_2$ | H | O | $3-CF_3$-phenyl | |
| 175 | " | " | " | " | 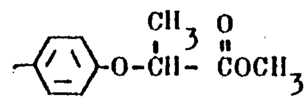 | 1,5654 |
| 176 | " | " | " | " | 2,4-Dichlorphenyl | |
| 177 | " | " | " | S | Phenyl | |
| 178 | " | " | " | " | 2,4-Dichlorphenyl | 1,6052 |
| 179 | " | " | " | " | $4-OCH_3$-phenyl | 1,5980 |
| 180 | " | " | " | " | 2-Carboxyphenyl | 157-161 |
| 181 | " | " | " | S=O | 2,4-dichlorphenyl | |
| 182 | " | " | " | $SO_2$ | 2,4-dichlorphenyl | |
| 186 | " | $NO_2$ | " | O | $COCH_3$ | 1,5454 |
| 187 | " | " | " | " | $COCH_2Cl$ | |
| 188 | " | " | " | " | $COCH_2OCH_3$ | |
| 189 | " | " | " | " | 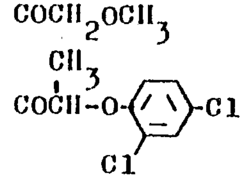 | 1,5669 |

| Verbindung Nr. | D | Y | $Z_4$ | X | A | Fp [°C] $n_D^{25}$, Wellenlänge einer Bande im IR-Spektrum |
|---|---|---|---|---|---|---|
| 190 | 2'-Chlor-4'-trifluormethylphenyl | $NO_2$ | H | O | CO-phenyl | |
| 191 | " | " | " | " | CO- 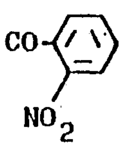 | |
| 192 | " | " | " | " | $SO_2CH_3$ | |
| 193 | " | " | " | " | $SO_2CF_3$ | |
| 194 | " | " | " | " | $SO_2NHCH_3$ | |
| 195 | " | " | " | " | $SO_2NH$-n-propyl | |
| 196 | " | " | " | " | $SO_2N$-$(CH_3)_2$ | |

| Verbindung Nr. | D | Y | $Z_4$ | X | A | Fp [°C] $n_D^{25}$, Wellenlänge einer Bande im IR-Spektrum |
|---|---|---|---|---|---|---|
| 203 | 2'-Chlor-4'-trifluormethylphenyl | $NO_2$ | H | O | $Si(CH_3)_3$ | 58- 62 |
| 204 | " | " | " | " | $Si(C_2H_5)_3$ | |
| 205 | " | " | " | " | Si $(CH_3)_2$<br>$C_4H_9$-n | |
| 209 | " | $NO_2$ | " | " | O<br>$P(OC_2H_5)_2$ | |
| 210 | " | " | " | S | S<br>$P(OCH_3)_2$ | P=S = 830 |
| 211 | " | " | " | " | O<br>P S-i-$C_3H_7$<br>$OC_2H_5$ | |
| 212 | " | " | " | " | O<br>P $OC_2H_5$<br>$N(CH_3)_2$ | P=O = 1260 |

| Verbindung Nr. | D | Y | $Z_4$ | X | A | Fp [°C] $n_D^{25}$, Wellenlänge einer Bande im IR-Spektrum |
|---|---|---|---|---|---|---|
| 214 | 2'-Chlor-4'-trifluormethylphenyl | $NO_2$ | O | O | $-(CH_2)_2$ | 1,5541 |
| 215 | " | " | S | S | $-(CH_2)_3$ | |

| Verbindung Nr. | D | Y | $Z_4$ | X | A | Fp [°C] $n_D^{25}$, Wellenlänge einer Bande im IR-Spektrum |
|---|---|---|---|---|---|---|
| 321 | 2'-Chlor-4'-trifluormethylphenyl | $NO_2$ | H | S | $CH_2COO-CH{<}^{CH_3}_{CH_3}$ | 66- 68 |
| 322 | " | " | " | " | $CH_2COONa$ | C=O = 1600 |
| 323 | " | " | " | " | $\underset{CH_3}{CH}-COOH$ | 1,5636 |
| 324 | " | " | " | " | $CH_2COOC_2H_5$ | 58- 61 |
| 325 | " | " | " | " | $CH_2CH_2COOC_{12}H_{25}$ | 1,5188 |
| 326 | " | " | " | " | $CH_2COOC_{18}H_{37}$ | C=O = 1720 |

| Verbindung Nr. | D | Y | $Z_H$ | X | A | Fp [°C] $n_D^{25}$, Wellenlänge einer Bande im IR-Spektrum |
|---|---|---|---|---|---|---|
| 333 | 2'-Chlor-4'-trifluormethylphenyl | $NO_2$ | H | S | $N(C_3H_7\text{-i-})_2$ | 1.5508 |
| 334 | " | " | " | " | $N<^{C_6H_{12}}_{C_3H_7(i)}$ | 1.5789 |

Die Anwendung der Wirkstoffe erfolgt z. B. in Form von direkt versprühbaren Lösungen, Pulvern, Suspensionen — auch hochprozentige wässerige, ölige oder sonstige Suspensionen — oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem mit hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z. B. Dimethylformid, Dimethylsulfoxid, N-Methylpyrrolidon, Wasser usw. in Betracht.

Wässerige Anwendungsformen können aus Emulsionskonzentraten, Pasten oder netzbaren Pulvern (Spritzpulvern, Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen : Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoleter, Kondensationsprodukte von sulfoniertem Naphthalin und Naphtalinderivaten mit Formaldehyd, Kondensationsprodukte des Naphthalins bzw. der Naphthalinsulfonsaüren mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

Die Wirkstoffe werden angewendet, beispielsweise durch Gießen, Streuen, Stäuben, Spritzen oder Sprühen auf die Pflanzen oder den Boden, durch Injizieren oder Bestreichen von Pflanzen oder durch Einbringen in das Bewässerungswasser.

Die Applikation kann im Vorauflaufverfahren oder bei Nachauflaufanwendung erfolgen. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,025 bis 15 kg/ha und mehr.

Der Einfluß von Vertretern der neuen herbiziden Aralkylanilinderivate auf das Wachstum von erwünschten und unerwünschten Pflanzen wird anhand von Gewächshausversuchen vorgeführt :

Als Kulturgefäße dienten Plastikblumentöpfe mit 300 $cm^3$ Inhalt und lehmigem Sand mit etwa 1,5 % Humus als Substrat. Die Samen der Testpflanzen wurden nach Arten getrennt flach eingesät. Unmittelbar danach erfolgte bei Vorauflaufbehandlung das Aufbringen der Wirkstoffe aud die Erdoberfläche. Sie wurden hierbei in Wasser als Verteilungsmittel suspendiert oder emulgiert und mittels fein verteilender Düsen gespritzt. Bei dieser Applikationsmethode handelt es sich um eine Aufwandmenge entsprechend 3,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde.

Zum Zwecke der Nachauflaufbehandlung zog man die Testpflanzen je nach Wuchsform erst bis zu

einer Wuchshöhe von 3 bis 15 cm an und behandelte sie danach. Die für die Nachauflaufanwendung benutzten Reispflanzen zog man im Gegensatz zu der übrigen Erde in einem mit Torfmull (peat) angereicherten Substrat an. Zur Nachauflaufbehandlung wurden entweder direkt gesäte und in den gleichen Gefäßen aufgewachsene Pflanzen ausgewählt, oder sie wurden erst als Keimpflanzen getrennt angezogen und einige Tage vor der Behandlung in die Versuchgefäße verpflanzt. Die Aufwandmengen für die Nachauflaufbehandlung variieren je nach Wirkstoff. Sie betrungen im Einzelfall 0,125, 0,25, 0,5 und auch bei einigen Verbindungen 3,0 kg Wirkstoff je ha.

Als Vergleichsmittel wurden folgende bekannte Verbindungen

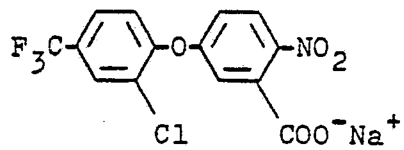

(A)

und

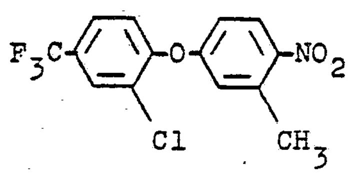

(B)

und

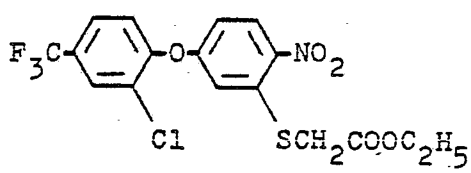

(C)

zu je 0,125 kg Wirkstoff/ha gewählt.

Eine Abdeckung unterblieb bei der Nachauflaufbehandlung. Die Aufstellung der Versuche erfolgte im Gewächshaus, wobei für wärmeliebende Arten wärmere Bereiche (20 bis 35 °C) und für solche gemäßigter Klimate 10 bis 25 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Bei der Prüfung der herbiziden Wirkung bei Vorauflaufanwendung im Gewächshaus bei Aufwandmengen von 3,0 kg Wirkstoff/ha zeigen die neuen Verbindungen Nr. 321, 322, 96, 10, 203, 11, 18, 175, 13, 94, 326, 15 eine sehr gute herbizide Wirkung.

Ebenfalls bei der Prüfung im Gewächshaus bei Nachauflaufanwendung waren die Wirkstoffe Nr. 5, 14, 148, 120, 153, 324, 321, 140, 142 mit 0,25 kg/ha und die Wirkstoffe Nr. 87, 13: 16, 101, 175, 214, 96, 104, 325 mit 0,5 kg Wirkstoff/ha sehr gut wirksam gegen breitblättrige unerwünschte Pflanzen.

Bei der Prüfung auf selektive herbizide Wirkung bei Nachauflaufanwendung im Gewächshaus zeigte der Wirkstoff Nr. 153 mit 0,125 kg/ha gegen einzelne breitblättrige Unkrautarten eine überlegene herbizide Aktivität und gleichzeitig eine bessere Verträglichkeit für Weizen mit höchstenfalls geringen temporären Blattverbrennungen, als die bekannte Vergleichssubstanz B in derselben Dosierung.

Die Gewächshausversuche ergaben ferner, das Verbindung Nr. 104 mit 0,125 kg Wirkstoff/ha im Nachauflaufverfahren angewandt eine bessere herbizide Wirkung hat als das Vergleichsmittel A und zugleich bei Weizen eine bessere Verträglichkeit besitzt.

Ebenso erbrachten die Gewächshausversuche, daß die Verbindungen Nr. 5 und 16 mit 0,125 kg Wirkstoff/ha bei Blattbehandlung eine ähnliche herbizide Aktivität haben wie das bekannte Vergleichsmittel C. Dafür sind jedoch die beiden neuen Verbindungen 5 und 16 verträglicher für Erdnüsse als die Vergleichsverbindung C und Verbindung Nr. 5 ist zudem noch verträglicher für Weizen.

Die neuen Verbindungen sind selektiv herbizid bei zahlreichen Kulturpflanzen. In Betracht kommen beispielsweise folgende Kulturen :

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor - Färberdistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |

| Botanischer Name | Deutscher Name | Englischer Name |
|---|---|---|
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glyoine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakatuschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |

| Botanischer Name | Deutscher Name | Englischer Name |
|---|---|---|
| Manihot esculenta | Maniok | cassava |
| Medicago sativa | Luzerne | alfalfa (lucerne) |
| Metha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |

| Botanischer Name | Deutscher Name | Englischer Name |
|---|---|---|
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sasamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn maize |

Zur Verbreiterung des Wirkungsspektrums und zur Erzielung synergistischer Effekte können die neuen Diphenyletherderivate sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht. Eine Reihe von Wirkstoffen, welche zusammen mit den neuen Verbindungen für verschiedenste Anwendungsbereiche sinnvolle Mischungen ergeben, werden beispielhaft aufgeführt :

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon

5-Methylamino-4-chlor-2-(3.trifluormethylphenyl)-3-(2H)-pyridazinon
5-Methylamino-4-chlor-2-(3.α,α,β,β-tetrafluorothoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3.trifluormethylphenyl)-3(2H)-pyridazinon
5-Methoxy-4-chlor-2-(3.trifluormethylphenyl)-3(2H)-pyridazinon
5-Amino-4-brom-2-(3.methylphenyl)-3(2H)-pyridazinon
3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze
1-Methoxymethyl-3-(1-methylethyl-2,1,3-benzothiadiazîn-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Cyan-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-Methylethyl)-1H-pyridino-[3,2-e] 2,1,3-thiadiazin-(4)-on-2,2-dioxid

N-(1-Ethylpropyl)-2,6-dinitro-3,4-dimethylanilin
N-(1-Methyläthyl)-N-ethyl-2,6-dinitro-4-trifluormethylanilin
N-n.Propyl-N-β-chlorethyl-2,6-dinitro-4-trifluormethylanilin
N-n.Propyl-N-cyclopropylmethyl-2,6-dinitro-4-trifluormethyl-anilin
N-Bis-(n.propyl)-2,6-dinitro-3-amino-4-trifluormethylanilin
N-Bis-(n.propyl)-2,6-dinitro-4-methyl-anilin
N-Bis-(n.propyl)-2,6-dinitro-4-methylsulfonyl-anilin
N-Bis-(n.propyl)-2,6-dinitro-4-aminosulfonyl-anilin
Bis-(β-chlorethyl)-2,6-dinitro-4-methyl-anilin
N-Ethyl-N-(2-methylallyl)-2,6-dinitro-4-trifluormethylanilin

N-Methylcarbaminsäure-3,4-dichlorbenzylester
N-Methylcarbaminsäure-2,6-di-tert.butyl-4-methylphenylester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'N-Isopropyl-carbamoyloxy-propionsäureanilid

Ethyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat

Methyl-N-(3-(N'-3-chlor-4-fluorphenylcarbamoyloxy)-phenyl)-carbamat
Ethyl-N-(3-(N'-3-chlor-4-fluorphenylcarbamoyloxy)-phenyl)-carbamat
Ethyl-N-(3-(N'-3,4-difluorphenylcarbamoyloxy)-phenyl)-carbamat
Methyl-N-(3-(N'-3,4-difluorphenylcarbamoyloxy)-phenyl)-carbamat

N-3-(4-Fluorphenoxycarbonylamino)-phenylcarbaminsäuremethylester
N-3-(2-Methylphenoxycarbonylamino)-phenylcarbaminsäureethylester
N-3-(4-Fluorphenoxycarbonylamino)-phenylthiolcarbaminsäuremethylester
N-3-(2,4,5-Trimethylphenoxycarbonylamino)-phenylthiolcarbaminsäure-methylester
N-3-(Phenoxycarbonylamino)-phenylthiolcarbaminsäuremethylester

N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolylmethylester
N,N-Di-isopropyl-thiolcarbaminsäure-3-ethyl-5-isoxazolylmethylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo-[2,2,1]-heptyl-thiolcarbaminsäureethylester
S-(2,3-Dichlorallyl)-(2,2,4-trimethyl-azetidin)-1-carbothiolat
S-(2,3,3-Trichlorallyl-(2,2,4-trimethyl-azetidin)-1-carbothiolat
S-Ethyl-hexahydro-1-H-azepin-1-carbothiolat
S-Benzyl-3-methylhexahydro-1-H-azepin-1-carbothiolat
S-Benzyl-2,3-dimethylhexahydro-1-H-azepin-1-carbothiolat
S-Ethyl-3-methylhexahydro-1-H-azepin-1-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
α,α-Dichlorpropionsäure-Natriumsalz
α,α-Dichlorbuttersäure-Natriumsalz
α,α,β,β-Tetrafluorpropionsäure-Natriumsalz
α-Methyl,α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure
2,3,5-Triiodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrechlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-(Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäuremethylester
2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäuremethylester
2-(N-Benzoyl-3-chlor-4-fluorphenylamino)-propionsäure-isopropylester

2-Chlor-4-ethylamino-6-isopropylamino-1,3,5-triazin
2-Chlor-4-ethylamino-6-(amino-2'-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin

2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisiisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethyluracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')-]-ethan (Salze)
1-(4-Chlorphenoxy-3,3-dimethyl-1(1H)-1,2,4-triazol-1-yl)-2-butanon
N,N-Diallylchloracetamid
N-Isopropyl-2-chloracetanilid
N-(1-Methyl-propin-2-yl)-2-chloracetanilid

2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid
2-Methyl-6-ethyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-(isobutoxymethyl)-2-chloracetanilid
2,6-Diethyl-N-(methoxymethyl)-2-chloracetanilid
2,6-Diethyl-N-(n-butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diethyl-N-(2-n-propoxyethyl)-2-chloracetanilid

2-(2-Methyl-4-chlorphenoxy)-N-methoxy-acetamid
2-(α-Naphtoxy)-N,N-diethylpropionamid
2,2-Diphenyl-N,N-dimethylacetamid
α-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonalinid
5-Acetamido-4-methyl-trifluormethan-sulfonanilid

2-Propionyl-amino-4-methyl-5-chlor-thiazol
O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)

Pentachlorphenol-Natriumsalz
2,4-Dichlorphenyl-4′-nitrophenylether
2,4,6-Trichlorphenyl-4′-nitrophenylether
2-Fluor-4,6-dichlorphenyl-4′-nitrophenylether
2-Chlor-4-trifluormethylphenyl-4′-nitrophenylether
2,4′-Dinitro-4-trifluormethyl-diphenylether
2,4-Dichlorphenyl-3′-methoxy-4′-nitro-phenylether
2-Chlor-4-trifluormethylphenyl-3′-ethoxy-4′-nitro-phenylether
2-Chlor-4-trifluormethylphenyl-3′-carboxy-4′-nitro-phenylether (Salze)
2,4-Dichlorphenyl-3′-methoxycarbonyl-4′-nitro-phenylether
2-(3,4-Dichlorphenyl-3′-methoxycarbonyl-4′-nitro-phenylether
2-(3-tert.Butylcarbamoyl-oxyphenyl)-4-methyl-1,2,4-oxadiazolidin-3,5-dion
2-(3-iso-Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methansulfonat
2-Ethoxy-2,3-dihydro-3,3-dimethyl-5-benzofuranyl-dimethylaminosulfonat
2-Ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)
2-sec.Butyl-4,6-dinitrophenol (Salze)
2-sec.Butyl-4,6-dinitrophenol-acetat
2-tert.Butyl-4,6-dinitrophenol-acetat

2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat

2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(3-α,α,β,β-Tetrafluorethoxyphenyl)-3,3-dimethyl-harnstoff

1-(3-tert.-Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4′-Chlorphenoxy)-phenyl]-3,3-dimethyl-harnstoff
1-[4(4′-methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff

1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethylharnstoff
Imidazolidin-2-on-1-carbonsäure-iso-butylamid
1,2-Dimethyl-3,5-diphenylpyrazolium-methylsulfat
1,2,4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenyl)-sulfonyl-oxyl]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridilium-di-(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino)-butyliden]-5,5-dimethylcyclohexan-1,3-dion (Salze)
2-[1-(N-Allyloxyamino)-butyliden]-5,5-dimethyl-4-methoxy-carbonyl-cyclohexan-1,3-dion (Salze)
2-Chlorphenoxyessigsäure (Salze, Ester, Amide)
4-Chlorphenoxyessgisäure (Salze, Ester, Amide)
2,4-Dichlorphenoxyessgsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridynyl-oxyessigsäure (Salze, Ester, Amide)
α-Naphtoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsaüre (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis-(phosphonmethyl)-glycin (Salze)
2-Chlorethanphosphonsäure-2-chlorethylester
Ammonium-ethyl-carbamoyl-phosphonat
Di-n-butyl-1-n-butylamino-cyclohexyl-phosphonat
Trithiobutylphosphit
O,O-Diisopropyl-5-(2-benzosulfonylamino-ethyl)-phosphordithioat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon

Natriumchlorat
Ammoniumrhodanid
Calciumcyanamid
2-Chlor-4-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert.Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Jodpyridyl-2-oxy)-phenoxy]-propionsäureethylester
2-[4-(5'-Jodpyridyl-2-oxy)-phenoxy]-propionsäure-n.-butylester
2-Chlor-4-trifluormethylphenyl-3'-(2-fluorethoxy)-4'-nitrophenyleter
2-Chlor-4-trifluormethylphenyl-3-(ethoxycarbonyl)methylthio-4-nitrophenylether
2,4,6-Trichlorphenyl-3(ethoxycarbonyl)methylthio-4-nitrophenylether
2-[1-(N-ethoxamino)-butyliden]-5-(2-ethylthiopropyl)-3-hydroxy-cyclohexen-(2)-on-(1) (Salze)
2-[1-(N-ethoxamino-butyliden]-5-(2-phenylthiopropyl)-3-hydroxy-cyclohexen-(2)-on-(1) (Salze)
4-[4-(4'-Trifluormethyl)-phenoxy]-penten-2-carbonsäureethylester
2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-α,α,β-Trifluor-β-bromethoxyphenyl)-3-(2H)-pyridazinon
2,4-Dichlorphenyl-3'-ethoxy-ethoxy-ethoxy-4'-nitrophenylether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1(3-Chlor-4-ethoxyphenyl)-3,3-dimethylharnstoff
2-Methyl-4-Chlorphenoxy-thioessigsäureethylester
2-Chlor-3,5-dijod-4-acetoxy-pyridin

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytophathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

**Patentansprüche** (für die Vertragsstaaten : BE, CH, DE, FR, GB, IT, LI, LU, NL, SE)

1. Substituierte Diphenylether der Formel

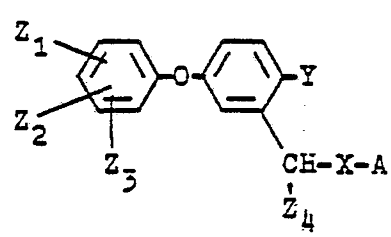

(I)

in der
$Z_1$ 2-Chlor,
$Z_2$ 4-Trifluormethyl, $Z_3$ Wasserstoff,
$Z_4$ Wasserstoff, Cyan, $C_1$-$C_4$ Alkyl,
$C_1$-$C_4$ Alkoxy, Acetoxy oder $C_1$-$C_4$ Alkylmercapto bedeutet,
Y Nitro,
X Sauerstoff, Schwefel, Sulfinyl oder Sulfonyl bedeutet,
und
A $C_1$-$C_6$ Alkyl, substituiertes $C_1$-$C_4$ Alkyl

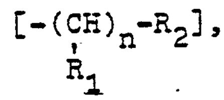

Tetrahydrofurfuryl,
alkyl ($C_1$-$C_4$)-substituiertes Tetrahydrofurfuryl,
Phenyl,
substituiertes Phenyl

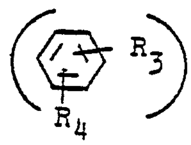

Benzyl,
substituiertes Benzyl

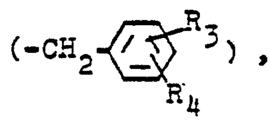

Acyl

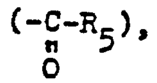

Silyl

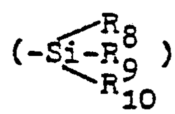

oder Phosphatyl

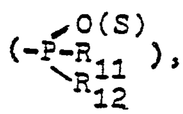

bedeutet, wobei im Falle X = Sauerstoff A zusätzlich Sulfonyl ($—SO_2R_{13}$) oder Sulfamoyl

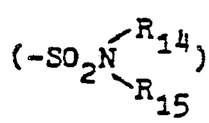

bedeutet und im Falle $Z_4$ = Alkoxy oder Alkylmercapto A zusätzlich eine Methylenkette $—(CH_2)_m—$ bedeutet, durch die die Reste $—Z_4—CH—X—$ zu einem Ring verbunden werden, $R_1$ Wasserstoff, Methyl, Ethyl oder n-Propyl, $R_2$ Cyan, Methoxy, Ethoxy oder

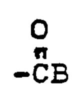

bedeutet, wobei B OH, ONa, $OCH_3$, $OC_2H_5$, $OC_3H_7$(i) oder O-Alkyl $C_4$-$C_{20}$, O-Phenyl, $—NH_2$, —NH Alkyl $C_1$-$C_4$, —N(Alkyl $C_1$-$C_4$)$_2$ bedeutet, n bedeutet 1, 2 oder 3, $R_3$ und $R_4$ bedeuten unabhängig voneinander Wasserstoff, Halogen, Methyl, Nitro, Cyan, Methoxy, Carboxyl, Trifluormethyl oder O-Propionsäuremethylester, $R_5$ bedeutet Methyl, Ethyl, Propyl, Chlormethyl, Dichlormethyl, Trichlormethyl, Methoxymethyl, Phenyl sowie Nitro- oder alkyl ($C_1$-$C_4$) substituiertes Phenyl oder α-2,4-Dichlorphenoxyethyl, $R_8$, $R_9$ und $R_{10}$ bedeuten Methyl, Ethyl, n-Propyl oder n-Butyl und können gleich oder verschieden sein, $R_{11}$ und $R_{12}$ bedeuten Methoxy, Ethoxy, Thiomethyl, Thioethyl, Thio-n-propyl, N,N-Dimethylamino oder N,N-Diethylamino und können gleich oder verschieden sein, $R_{13}$ bedeutet Methyl, Ethyl, Phenyl oder Trifluormethyl, $R_{14}$ und $R_{15}$ bedeuten Wasserstoff, Methyl, Ethyl, n-Propyl, iso-Propyl, n-Butyl, n-Hexyl, n-Heptyl, Methoxy, Ethoxy oder Isopropyloxy und können gleich oder verschieden sein, m bedeutet 2 oder 3.

2. Herbizid, enthaltend einen substituierten Diphenylether der Formel I gemäß Anspruch 1.

3. Herbizid, enthaltend einen festen oder flüssigen Trägerstoff und einen substituierten Diphenylether der Formel I gemäß Anspruch 1.

4. Verfahren zur Herstellung eines Herbizids, dadurch gekennzeichnet, daß man einen festen oder flüssigen Trägerstoff vermischt mit einem substituierten Diphenylether der Formel I gemäß Anspruch 1.

5. Verfahren zur Bekämpfung von unerwünschten Pflanzen, dadurch gekennzeichnet, daß man die Pflanzen oder den Boden behandelt mit einem substituierten Diphenylether der Formel I gemäß Anspruch 1.

6. Substituierte Diphenylether, ausgewählt aus der Gruppe, bestehend aus 3-(2'-Chlor-4'-trifluormethylphenoxy)-6-nitro-benzyloxyessigsäuremethylester, 3-(2'-Chlor-4'-trifluormethylphenoxy)-6-nitro-benzyloxy-N-isopropylamidosulfonat, 3-(2'-Chlor-4'-trifluormethylphenoxy)-6-nitro-benzyl-thiolessigsäuremethylester, 3-(2'-Chlor-4'-trifluormethylphenoxy)-6-nitro-benzyl-sulfonessigsäuremethylester.

7. Herbizid, enthaltend einen substituierten Diphenylether ausgewählt aus der Gruppe bestehend aus 3-(2'-Chlor-4'-trifluormethylphenoxy)-6-nitro-benzyloxyessigsäuremethylester, 3-(2'-Chlor-4'-trifluormethylphenoxy)-6-nitro-benzyloxy-N-isopropylamidosulfonat, 3-(2'-Chlor-4'-trifluormethylphenoxy)-6-nitro-benzyl-thiolessigsäuremethylester, 3-(2'-Chlor-4'-trifluormethylphenoxy)-6-nitro-benzyl-sulfonessigsäuremethylester.

**Patentansprüche** (für den Vertragsstaat AT)

1. Herbizid, enthaltend einen substituierten Diphenylether der Formel

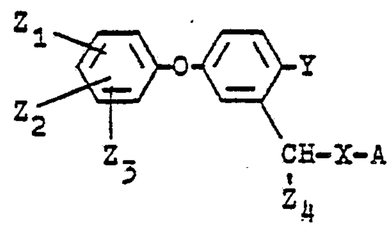

(I)

in der

$Z_1$ 2-Chlor,
$Z_2$ 4-Trifluormethyl, $Z_3$ Wasserstoff,
$Z_4$ Wasserstoff, Cyan, $C_1$-$C_4$ Alkyl,
$C_1$-$C_4$ Alkoxy, Acetoxy oder $C_1$-$C_4$ Alkylmercapto bedeutet,
Y Nitro,
X Sauerstoff, Schwefel, Sulfinyl oder Sulfonyl bedeutet,

und

A $C_1$-$C_6$ Alkyl,
substituiertes $C_1$-$C_4$ Alkyl

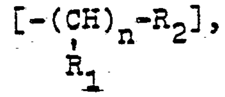

Tetrahydrofurfuryl,
alkyl ($C_1$-$C_4$)- substituiertes Tetrahydrofurfuryl,
Phenyl,
substituiertes Phenyl

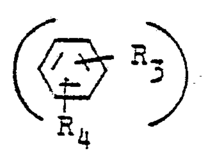

Benzyl,
substituiertes Benzyl

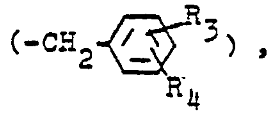

Acyl

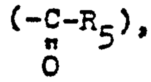

Silyl

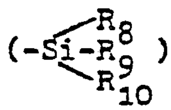

oder Phosphatyl

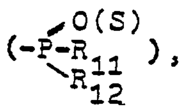

bedeutet, wobei im Falle X = Sauerstoff A zusätzlich Sulfonyl ($—SO_2R_{13}$) oder Sulfamoyl

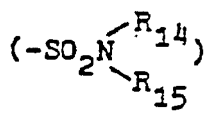

bedeutet und im Falle $Z_4$ = Alkoxy oder Alkylmercapto A zusätzlich eine Methylenkette $—(CH_2)_m—$ bedeutet, durch die die Reste $—Z_4—CH—X—$ zu einem Ring vebunden werden, $R_1$ Wasserstoff, Methyl, Ethyl oder n-Propyl, $R_2$ Cyan, Methoxy, Ethoxy oder

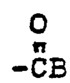

bedeutet, wobei B OH, ONa, $OCH_3$, $OC_2H_5$, $OC_3H_7$(i) oder O-Alkyl $C_4$-$C_{20}$, O-Phenyl, $—NH_2$, NH-Alkyl $C_1$-$C_4$, —N(Alkyl $C_1$-$C_4$)$_2$ bedeutet, n bedeutet 1, 2 oder 3, $R_3$ und $R_4$ bedeuten unabhängig voneinander Wasserstoff, Halogen, Methyl, Nitro, Cyan, Methoxy, Carboxyl, Trifluormethyl oder O-Propionsäuremethylester, $R_5$ bedeutet Methyl, Ethyl, Propyl, Chlormethyl, Dichlormethyl, Trichlormethyl, Methoxymethyl, Phenyl sowie Nitro- oder alkyl($C_1$-$C_4$) substituiertes Phenyl oder α-2,4-Dichlorphenoxyethyl, $R_8$, $R_9$ und $R_{10}$ bedeuten Methyl, Ethyl, n-Propyl oder n-Butyl und können gleich oder verschieden sein, $R_{11}$ und $R_{12}$ bedeuten Methoxy, Ethoxy, Thiomethyl, Thioethyl, Thio-n-propyl, N,N-Dimethylamino oder N,N-Diethylamino und können gleich oder verschieden sein, $R_{13}$ bedeutet Methyl, Ethyl, Phenyl oder Trifluormethyl, $R_{14}$ und $R_{15}$ bedeuten Wasserstoff, Methyl, Ethyl, n-Propyl, iso-Propyl, n-Butyl, n-Hexyl, n-Heptyl, Methoxy, Ethoxy oder Isopropyloxy und können gleich oder verschieden sein, m bedeutet 2 oder 3.

2. Herbizid, enthaltend einen festen oder flüssigen Trägerstoff und einen substituierten Diphenylether der Formel I gemäß Anspruch 1.

3. Verfahren zur Herstellung eines Herbizids, dadurch gekennzeichnet, daß man einen festen oder flüssigen Trägerstoff vermischt mit einem substituierten Diphenylether der Formel I gemäß Anspruch 1.

4. Verfahren zur Bekämpfung von unerwünschten Pflanzen, dadurch gekennzeichnet, daß man die Pflanzen oder den Boden behandelt mit einem substituierten Diphenylether der Formel I gemäß Anspruch 1.

5. Herbizid, enthaltend einen substituierten Diphenylether ausgewählt aus der Gruppe bestehend aus 3-(2'-Chlor-4'-trifluormethylphenoxy)-6-nitro-benzyloxyessigsäuremethylester, 3-(2'-Chlor-4'-trifluormethylphenoxy)-6-nitro-benzyloxy-N-isopropylamidosulfonat, 3-(2'-Chlor-4'-trifluormethylphenoxy)-6-nitro-benzyl-thiolessigsäuremethylester, 3-(2'-Chlor-4'-trifluormethylphenoxy)-6-nitro-benzyl-sulfonessigsäuremethylester.

**Claims** (for the Contracting States : BE, CH, DE, FR, GB, IT, LI, LU, NL, SE)

1. A substituted diphenyl ether of the formula

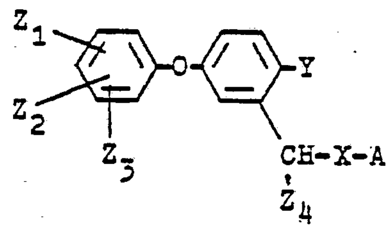

(I)

where

$Z_1$ is 2-chloro, $Z_2$ is 4-trifluoromethyl, $Z_3$ is hydrogen,
$Z_4$ is hydrogen, cyano, $C_1$-$C_4$-alkyl,
$C_1$-$C_4$-alkoxy, acetoxy or $C_1$-$C_4$-alkylmercapto,
Y is nitro,
X is oxygen, sulfur, sulfinyl or sulfonyl,

and

A is $C_1$-$C_6$-alkyl, substituted $C_1$-$C_4$-alkyl

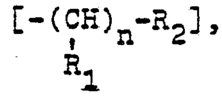

tetrahydrofurfuryl, $C_1$-$C_4$-alkyl-substituted tetrahydrofurfuryl, phenyl, substituted phenyl

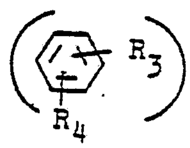

benzyl, substituted benzyl

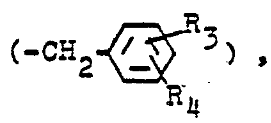

acyl

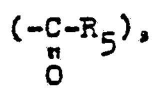

silyl

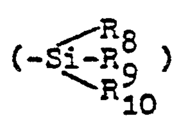

or phosphatyl

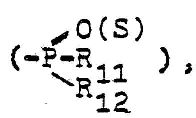

and can also be sulfonyl ($—SO_2R_{13}$) or sulfamyl

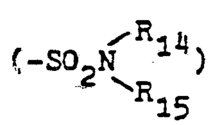

when X is oxygen, and, when $Z_4$ is alkoxy or alkylmercapto, A can also be a methylene chain $—(CH_2)_m—$ by which the radicals $Z_4$—CH—X— are bonded to form a ring, $R_1$ is hydrogen, methyl, ethyl or n-propyl, $R_2$ is cyano, methoxy, ethoxy or

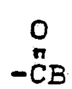

where B is OH, ONa, $OCH_3$, $OC_2H_5$, O-i-$C_3$-$H_7$ or O-($C_4$-$C_{20}$)-alkyl, O-phenyl, —$NH_2$, —NH($C_1$-$C_4$)-alkyl or N($C_1$-$C_4$-alkyl)$_2$, n is 1, 2 or 3, $R_3$ and $R_4$ independently of one another are hydrogen, halogen, methyl, nitro, cyano, methoxy, carboxyl, trifluoromethyl or an O-propionic acid methyl ester group, $R_5$ is methyl, ethyl, propyl, chloromethyl, dichloromethyl, trichloromethyl, methoxymethyl, phenyl, nitro- or $C_1$-$C_4$-alkyl-substituted phenyl or α-2,4-dichlorophenoxyethyl, $R_8$, $R_9$ and $R_{10}$ are each methyl, ethyl, n-propyl or n-butyl and can be identical or different, $R_{11}$ and $R_{12}$ are each methoxy, ethoxy, thiomethyl, thioethyl, thio-n-propyl, N,N-dimethylamino or N,N-diethylamino and can be identical or different, $R_{13}$ is methyl, ethyl, phenyl or trifluoromethyl, $R_{14}$ and $R_{15}$ are each hydrogen, methyl, ethyl, n-propyl, iso-propyl, n-butyl, n-hexyl, n-heptyl, methoxy, ethoxy or isopropoxy and can be identical or different, and m is 2 or 3.

2. A herbicide containing a substituted diphenyl ether of the formula I as claimed in claim 1.

3. A herbicide containing a solid or liquid carrier and a substituted diphenyl ether of the formula I as claimed in claim 1.

4. A process for producing a herbicide, wherein a solid or liquid carrier is mixed with a substituted diphenyl ether of the formula I as claimed in claim 1.

5. A process for combating unwanted plants, wherein the plants or the soil are treated with a substituted diphenyl ether of the formula I as claimed in claim 1.

6. A substituted diphenyl ether selected from the group consisting of methyl 3-(2'-chloro-4'-trifluoromethylphenoxy)-6-nitrobenzoxyacetate, 3-(2'-chloro-4'-trifluoromethylphenoxy)-6-nitrobenzoxy-N-isopropylamidosulfonate, methyl 3-(2'-chloro-4'-trifluoromethylphenoxy)-6-nitrobenzylthioacetate and methyl 3-(2'-chloro-4'-trifluoromethylphenoxy)-6-nitrobenzylsulfoacetate.

7. A herbicide containing a substituted diphenyl ether selected from the group consisting of methyl 3-(2'-chloro-4'-trifluoromethylphenox)-6-nitrobenzoxyacetate, 3-(2'-chloro-4'-trifluoromethylphenoxy)-6-nitrobenzoxy-N-isopropylamidosulfonate, methyl 3-(2'-chloro-4'-trifluoromethylphenoxy)-6-nitrobenzylthioacetate and methyl 3-(2'-chloro-4'-trifluoromethylphenoxy)-6-nitrobenzylsulfoacetate.

**Claims** (for the Contracting State AT)

1. A herbicide containing a substituted diphenyl ether of the formula

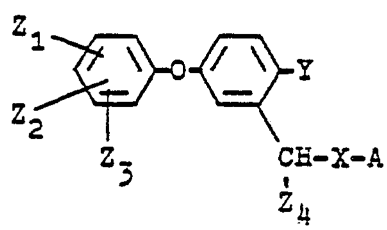

(I)

where

$Z_1$ is 2-chloro, $Z_2$ is 4-trifluoromethyl, $Z_3$ is hydrogen,
$Z_4$ is hydrogen, cyano, $C_1$-$C_4$-alkyl,
$C_1$-$C_4$-alkoxy, acetoxy or $C_1$-$C_4$-alkylmercapto,
Y is nitro,
X is oxygen, sulfur, sulfinyl or sulfonyl,

and

A is $C_1$-$C_6$-alkyl, substituted $C_1$-$C_4$-alkyl

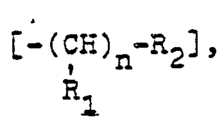

tetrahydrofurfuryl, $C_1$-$C_4$-alkyl-substituted tetrahydrofurfuryl, phenyl, substituted phenyl

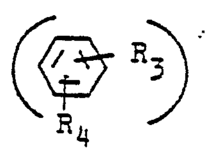

benzyl, substituted benzyl

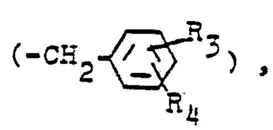

acyl

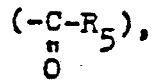

silyl

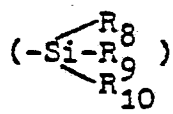

or phosphatyl

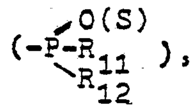

and can also be sulfonyl ($-SO_2R_{13}$) or sulfamyl

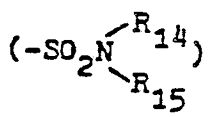

when X is oxygen, and, when $Z_4$ is alkoxy or alkylmercapto, A can also be a methylene chain $-(CH_2)_m-$ by which the radicals $Z_4-CH-X-$ are bonded to form a ring, $R_1$ is hydrogen, methyl, ethyl or n-propyl, $R_2$ is cyano, methoxy, ethoxy or

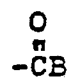

where B is OH, ONa, $OCH_3$, $OC_2H_5$, O-i-$C_3$-$H_7$ or O-($C_4$-$C_{20}$)-alkyl, O-phenyl, $-NH_2$, $-NH(C_1$-$C_4)$-alkyl or $-N(C_1$-$C_4$-alkyl$)_2$, n is 1, 2 or 3, $R_3$ and $R_4$ independently of one another are hydrogen, halogen, methyl, nitro, cyano, methoxy, carboxyl, trifluoromethyl or an O-propionic acid methyl ester group, $R_5$ is methyl, ethyl, propyl, chloromethyl, dichloromethyl, trichloromethyl, methoxymethyl, phenyl, nitro- or $C_1$-$C_4$-alkyl-substituted phenyl or α-2,4-dichlorophenoxyethyl, $R_8$, $R_9$ and $R_{10}$ are each methyl, ethyl, n-propyl or n-butyl and can be identical or different, $R_{11}$ and $R_{12}$ are each methoxy, ethoxy, thiomethyl, thioethyl, thio-n-propyl, N,N-dimethylamino, or N,N-diethylamino and can be identical or different, $R_{13}$ is methyl, ethyl, phenyl or trifluoromethyl, $R_{14}$ and $R_{15}$ are each hydrogen, methyl, ethyl, n-propyl, isopropyl, n-butyl, n-hexyl, n-heptyl, methoxy, ethoxy or isopropoxy and can be identical or different, and m is 2 or 3.

2. A herbicide containing a solid or liquid carrier and a substituted diphenyl ether of the formula I, as defined in claim 1.

3. A process for producing a herbicide, wherein a solid or liquid carrier is mixed with a substituted diphenyl ether of the formula I, as defined in claim 1.

4. A process for combating unwanted plants, wherein the plants or the soil are treated with a substituted diphenyl ether of the formula I, as defined in claim 1.

5. A herbicide containing a substituted diphenyl ether selected from the group consisting of methyl 3-(2'-chloro-4'-trifluoromethylphenoxy)-6-nitrobenzoxyacetate, 3-(2'-chloro-4'-trifluoromethylphenoxy)-6-nitrobenzoxy-N-isopropylamidosulfonate, methyl 3-(2'-chloro-4'-trifluoromethylphenoxy)-6-nitrobenzylthioacetate and methyl 3-(2'-chloro-4'-trifluoromethylphenoxy)-6-nitrobenzylsulfoacetate.

**Revendications** (pour les Etats contractants : BE, CH, DE, FR, GB, IT, LI, LU, NL, SE)

1. Diphényléthers substitués de formule

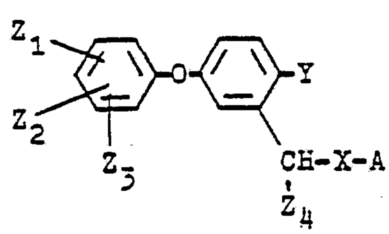

(I)

dans laquelle

$Z_1$ représente 2-chlore, $Z_2$ représente 4-trifluorométhyle, $Z_3$ représente hydrogène, $Z_4$, hydrogène, cyano, alkyle en $C_1$-$C_4$, alcoxy en $C_1$-$C_4$, acétoxy ou alkylmercapto en $C_1$-$C_4$,

Y représente nitro,

X oxygène, soufre, sulfinyle ou sulfonyle,

et

A représente alkyle en $C_1$-$C_6$, alkyle (en $C_1$-$C_4$)

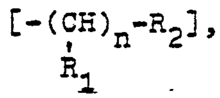

substitué,

tétrahydrofurfuryle, alkyle ($C_1$-$C_4$)-tétrahydrofurfuryle substitué, phényle, phényle

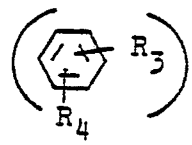

substitué, benzyle,

benzyle

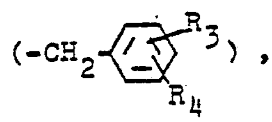

substitué,

acyle

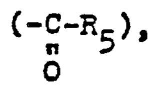

silyle

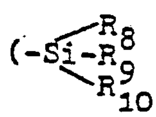

ou phosphatyle

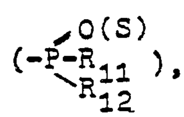

A représentant, en outre, sulfonyle ($—SO_2R_{13}$) ou sulfamoyle

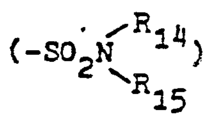

lorsque X = oxygène, et A représentant, en outre, lorsque $Z_4$ = alcoxy ou alkylmercapto, une chaîne méthylène $—(CH_2)_m—$, par laquelle les restes —$Z_4$—CH—X— sont reliés pour former une chaîne, $R_1$ représente hydrogène, méthyle, éthyle ou n-propyle, $R_2$ représente cyano, méthoxy, éthoxy ou

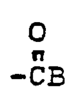

B représentant OH, ONa, $OCH_3$, $OC_2H_5$, $OC_3H_7(i)$ ou O-alkyle en $C_4$-$C_{20}$, O-phényle, $—NH_2$, —NH alkyle en $C_1$-$C_4$, —N' (alkyle en $C_1$-$C_4$)$_2$, n représente 1, 2 ou 3, $R_3$ et $R_4$ représentent, indépendamment l'un de l'autre, hydrogène, halogène, méthyle, nitro, cyano, méthoxy, carboxyle, trifluorométhyle ou O-propionate de méthyle, $R_5$ représente méthyle, éthyle, propyle, chlorométhyle, dichlorométhyle, trichlorométhyle, méthoxyméthyle, phényle ainsi que phényle substitué par nitro ou alkyle en $C_1$-$C_4$, ou α-2,4-dichlorophénoxyéthyle, $R_8$, $R_9$ et $R_{10}$ représentent méthyle, éthyle, n-propyle ou n-butyle et peuvent être identiques ou différents, $R_{11}$ et $R_{12}$ représentent méthoxy, éthoxy, thiométhyle, thioéthyle, thio-n-propyle, N,N-diméthylamino ou N,N-diéthylamino et peuvent être identiques ou différents, $R_{13}$ représente méthyle, éthyle, phényle ou trifluorométhyle, $R_{14}$ et $R_{15}$ représentent hydrogène, méthyle, éthyle, n-propyle, iso-propyle, n-butyle, n-hexyle, n-heptyle, méthoxy, éthoxy ou isopropyloxy et peuvent être identiques ou différents, m représente 2 ou 3.

2. Herbicide contenant un diphényléther substitué de formule I selon la revendication 1.

3. Herbicide contenant un support solide ou liquide et un diphényléther substitué de formule I selon la revendication 1.

4. Procédé de préparation d'un herbicide, caractérisé par le fait qu'on mélange un support solide ou liquide avec un diphényléther substitué de formule I selon la revendication 1.

5. Procédé de lutte contre les plantes indésirables, caractérisé par le fait que l'on traite les plantes ou le sol avec un diphényléther substitué de formule I selon la revendication 1.

6. Diphényléthers substitués choisis dans le groupe constitué par : 3-(2'-chloro-4'-trifluorométhylphénoxy)-6-nitrobenzyloxy acétate de méthyle, 3-(2'-chloro-4'-trifluorométhylphénoxy)-6-nitro-benzyloxy-N-isopropylamidosulfonate, 3-(2'-chloro-4'-trifluorométhylphénoxy)-6-nitro-benzyl-thiolacétate de méthyle, 3-(2'-chloro-4'-trifluorométhylphénoxy)-6-nitro-benzyl-sulfonacétate de méthyle.

7. Herbicide contenant un diphényléther substitué choisi dans le groupe constitué par : 3-(2'-chloro-4'-trifluorométhylphénoxy)-6-nitro-benzyloxy-acétate de méthyle, 3-(2'-chloro-4'-trifluorométhylphénoxy)-6-nitro-benzyloxy-N-isopropylamidosulfonate, 3-(2'-chloro-4'-trifluorométhylphénoxy)-6-nitro-benzyl-thiol-acétate de méthyle, 3-(2'-chloro-4'-trifluorométhylphénoxy)-6-nitro-benzyl-sulfonacétate de méthyle.

**Revendications** (pour l'Etat contractant AT)

1. Herbicide contenant un diphényléther substitué de formule

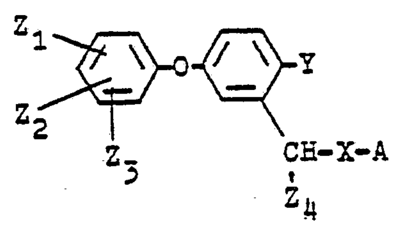

(I)

dans laquelle

$Z_1$ représente 2-chlore, $Z_2$ représente 4-trifluorométhyle, $Z_3$ représente hydrogène,
$Z_4$ hydrogène, cyano, alkyle en $C_1$-$C_4$, alcoxy en $C_1$-$C_4$, acétoxy ou alkylmercapto en $C_1$-$C_4$,
Y représente nitro,
X oxygène, soufre, sulfinyle ou sulfonyle,

et

A représente alkyle en $C_1$-$C_6$, alkyle (en $C_1$-$C_4$)

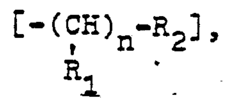

substitué,

tétrahydrofurfuryle, alkyle ($C_1$-$C_4$)-tétrahydrofurfuryle substitué, phényle,
phényle

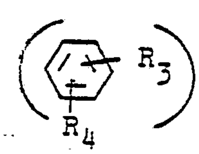

substitué, benzyle, benzyle

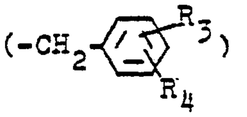 ,

substitué, acyle

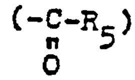,

silyle

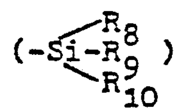

ou phosphatyle

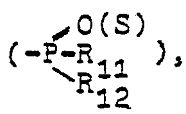,

A représentant en outre sulfonyle ($—SO_2R_{13}$) ou sulfamoyle

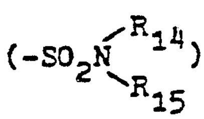

lorsque X = oxygène, et A représentant, en outre, lorsque $Z_4$ = alcoxy ou alkylmercapto, une chaîne méthylène $—(CH_2)_m—$, par laquelle les restes $—Z_4—CH—X—$ sont reliés pour former une chaîne, $R_1$ représente hydrogène, méthyle, éthyle ou n-propyle, $R_2$ représente cyano, méthoxy, éthoxy ou

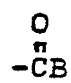

B représentant OH, ONa, $OCH_3$, $OC_2H_5$, $OC_3H_7$(i) ou O-alkyle en $C_4$-$C_{20}$, O-phényle, $—NH_2$, —NH alkyle en $C_1$-$C_4$, —N' (alkyle en $C_1$-$C_4$)$_2$, n représente 1, 2 ou 3, $R_3$ et $R_4$ représentent, indépendamment l'un de l'autre, hydrogène, halogène, méthyle, nitro, cyano, méthoxy, carboxyle, trifluorométhyle ou O-propionate de méthyle, $R_5$ représente méthyle, éthyle, propyle, chlorométhyle, dichlorométhyle, trichlorométhyle, méthoxyméthyle, phényle ainsi que phényle substitué par nitro ou alkyle en $C_1$-$C_4$, ou α-2,4-dichlorophénoxyéthyle, $R_8$, $R_9$ et $R_{10}$ représentent méthyle, éthyle, n-propyle ou n-butyle et peuvent être identiques ou différents, $R_{11}$ et $R_{12}$ représentent méthoxy, éthoxy, thiométhyle, thioéthyle, thio-n-propyle, N,N-diméthylamino ou N,N-diéthylamino et peuvent être identiques ou différents, $R_{13}$ représente méthyle, éthyle, phényle ou trifluorométhyle, $R_{14}$ et $R_{15}$ représentent hydrogène, méthyle, éthyle, n-propyle, iso-propyle, n-butyle, n-hexyle, n-heptyle, méthoxy, éthoxy ou isopropyloxy et peuvent être identiques ou différents, m représente 2 ou 3.

2. Herbicide contenant un support solide ou liquide et un diphényléther substitué de formule I selon la revendication 1.

3. Procédé de préparation d'un herbicide, caractérisé par le fait qu'on mélange un support solide ou liquide avec un diphényléther substitué de formule I selon la revendication 1.

4. Procédé de lutte contre les plantes indésirables, caractérisé par le fait que l'on traite les plantes ou le sol avec un diphényléther substitué de formule I selon la revendication 1.

5. Herbicide contenant un diphényléther substitué choisi dans le groupe constitué par : 3-(2'-chloro-4'-trifluorométhylphénoxy)-6-nitro-benzyloxy- acétate de méthyle, 3-(2'-chloro-4'-trifluorométhylphénoxy)-6-nitro-benzyloxy-N-isopropylamidosulfonate, 3-(2'-chloro-4'-trifluorométhylphénoxy)-6-nitro-benzyl-thiol-acétate de méthyle, 3-(2'-chloro-4'-trifluorométhylphénoxy)-6-nitro-benzyl-sulfonacétate de méthyle.